# EUROPÄISCHE PATENTSCHRIFT

(11) **EP 2 489 349 B1**
(45) Veröffentlichungstag und Bekanntmachung des Hinweises auf die Patenterteilung: **28.05.2014**
(21) Anmeldenummer: 11175546.8
(22) Anmeldetag: 27.07.2011
(51) Int. Cl.: A61K 9/50, A61K 9/16, A61K 38/46, A61K 38/54, C12N 9/94

(54) **Pankreatin-Pellets, insbesondere Pankreatin-Mikropellets, und Verfahren zu deren Herstellung**
Pancreatin pellets, in particular pancreatin micropellets and method for producing same
Granules de pancréatine, notamment micro-granules de pancréatine et leur procédé de fabrication

(30) Priorität: 30.03.2011 DE 202011000728 U; 17.02.2011 EP 11154829; 22.03.2011 EP 11159267; 31.05.2011 EP 11168323
(43) Veröffentlichungstag der Anmeldung: 22.08.2012
(73) Patentinhaber: Nordmark Arzneimittel GmbH & Co. KG, 25436 Uetersen (DE)
(72) Erfinder: Doleschal, Dr. Walter, 25436 Uetersen (DE); Kurfürst, Dr. Manfred, 25436 Moorrege (DE); Moest, Dr. Thomas, 25436 Moorrege (DE)
(74) Vertreter: Richter, Joachim

(56) Entgegenhaltungen:
- DE-U1-202010 004 926

## Beschreibung

Die Erfindung betrifft Pankreatin-Pellets, insbesondere Pankreatin-Mikropellets, und Verfahren zu deren Herstellung.

Als Pankreatin bezeichnet man das extrahierte Gemisch der Enzyme der Pankreasdrüse, bestehend im Wesentlichen aus Lipasen, Amylase und Proteasen. Als Ausgangsmaterial für die Herstellung von Pankreatin dient hauptsächlich Schweinepankreas in frischem oder gefrorenem Zustand, dem ursprünglich für die Herstellung von Pankreatin lediglich das Wasser und das Fett entzogen wurden. Im Hinblick auf die Empfindlichkeit der Enzyme sind jedoch Verfahren entwickelt worden, die eine möglichst schonende Gewinnung des Pankreatin ermöglichen sollen. Ein geeignetes Verfahren wird in DE 32 48 588 A1 beschrieben.

Pankreatin wird als Wirkstoff insbesondere zur Behandlung von Verdauungsstörungen, die auf Pankreasinsuffizienz beruhen, angewendet. Pankreatin wird hautsächlich in getrockneter Form als orales Therapeutikum verwendet. Dabei hat sich herausgestellt, dass die therapeutische Wirksamkeit der Pankreatingabe verbessert werden kann, wenn der Wirkstoff in Form von Pellets oder Mikropellets verabreicht wird.

Typischerweise werden Pankreatin-Pellets hergestellt, indem das Pankreatin mit Hilfsstoffen und Bindemitteln versetzt und diese Komponenten vermischt werden, bis ein homogenes Gemisch erhalten wird. Das homogene Gemisch wird in einen Extruder eingebracht, wo das Gemisch in ein strangförmiges Extrudat überführt wird. Das Extrudat wird schließlich in einen Spheronizer, gegebenenfalls unter Zusatz weiterer Hilfsstoffe eingebracht, in dem die Stränge unter Erhalt von kugelförmigen Pellets umgeformt werden. Die Pellets werden anschließend getrocknet und gesiebt, wobei Siebfraktionen mit Pellets, die einen vorgegebenen Größenbereich über- oder untersteigen, ausgesondert werden. Die so erhaltenen Pellets können anschließend mit einem Überzug aus einem magensaftresistenten Material lackiert werden.

Pankreatin fällt bei der Herstellung lösungsmittelfeucht an und wird dann normalerweise getrocknet und gemahlen. Nachteilig ist es nun, wenn dieses Material zur Formgebung noch mehrere Male, z. B. bei der Granulierung und der Rundung, befeuchtet und getrocknet werden muss. Dies verbraucht Zeit und Energie und führt oft zu Qualitätsverlusten durch Schädigung der hochempfindlichen Enzyme.

Bisher werden Pankreatin-Pellets durch Feuchtgranulierung aus trockenem Pankreatin-Pulver hergestellt. Üblicherweise werden hierzu noch Hilfs- und Klebestoffe benötigt. Ein Verfahren zur Herstellung von Panreatin-Pellets besteht darin, dass pulverförmiges Pankreatin mit einem Lösungsmittelgemisch angefeuchtet und mit einer Strangpresse zu zylindrischen Rohpellets geformt wird. Dazu müssen die Stränge dann noch auf eine Länge von beispielsweise 1,5 bis 1,7 mm abgeschnitten werden. Nach der Trocknung werden diese Rohpellets mit einem feuchten Pankreatin-/Lack-Gemisch ausgerundet und wiederum getrocknet.

Ein weiteres Verfahren zur Herstellung von Pankreatin-Mikropellets wird beispielsweise in EP 0 583 726 A2 beschrieben. Gemäß diesem Verfahren werden vor der Extrusion 100 Gewichtsteile Pankreatin mit 15 bis 50 Gewichtsteilen Polyethylenglykol 4000 und 10 bis 30 Gewichtsteilen eines Alkohols vermischt. Der Alkohol, beispielweise Propanol, soll dem Gemisch eine extrudierbare Konsistenz verleihen. Die mittels der Extrusion erhaltenen Stränge werden vor der Überführung in den Spheronizer mit 1,5 bis 5 Gewichtsteilen Paraffin und weiteren 1,5 bis 10 Gewichtsteilen Alkohol versetzt. Die erhaltenen Pellets besitzen einen Pankreatingehalt von 65 bis 85 Gew.-%, so dass die Pellets wenigstens 15 Gew.-% Hilfsstoffe und Bindemittel enthalten.

Die zur Extrusion erforderlichen Hilfsstoffe und Bindemittel können jedoch unerwünschte Nebenwirkungen haben. Aus diesem Grunde wird die Auswahl der Hilfsstoffe und Bindemittel einer ständigen Überprüfung unterzogen. Beispielsweise hat sich herausgestellt, dass der bislang übliche Zusatz mineralischer Öle nicht mehr als unkritisch angesehen werden kann.

Aus diesem Grunde wurde in EP 1 931 317 B1 ein Verfahren vorgeschlagen, mit dem es möglich sein soll, Pankreatin-Pellets ohne den Zusatz von Paraffin herzustellen. Die dort erhaltenen Pankreatin-Pellets enthalten 10 bis 95 Gew.-% Pankreatin, wobei zumindest 5 Gew.-% Hilfsstoffe und Bindemittel wie Polyethylenglycol sind.

Es ist jedoch zu erwarten, dass gegen Zusätze jeder Art begründete oder auch unbegründete Bedenken erhoben werden. Es ist daher wünschenswert, ein Pankreatin in einer Form bereitzustellen, die zum einen oral verabreicht werden kann und zum anderen so weitgehend wie möglich, frei von irgendwelchen Zusätzen ist. Mit einer 100 %igen Pankreatinkonzentration wird auch ein minimales Applikationsvolumen bei vorgegebener Dosis erreicht; dies erleichtert die Einnahme für den Patienten.

Die US 4,280,971 A1 offenbart ein Verfahren zur Herstellung von Pankreatin-Pellets, nach dem ein Pankreatin-Pulver und enzymfreundliche Lösungsmittel enthaltende verformbare Masse auf eine Strangpresse, gegebenen falls unter Kühlung, extrudiert, das Extrudat in Strangschnittlinge zerteilt, trocknet und nach bekannten Methoden weiterverarbeitet, z. B. durch Aufbringen von Überzügen auf die Strangschnittlinge. Die Verfahrensweise sieht jedoch zwei Trocknungsstufen vor, was unwirtschaftlich ist, denn es wird eine Mischung aus Magnesiumstearat, Pankreatin und Isopropanol hergestellt, die auf einer Strangpresse mit Bohrungen und einer Schneidevorrichtung zur Erzeugung von Strängen mit vorgegebener Länge verarbeitet werden. Die so erhaltenen Rohpellets werden in einer ersten Stufe getrocknet. Danach werden die Rohpellets durch Auftragen einer isopropanolischen Lösung von Polyvinylpyrrolidon und Pankreatin zu sphärisch geformten Pellets ausgerundet, die in einer zweiten Stufe einer weiteren Trocknung unterzogen werden. Bei diesem Verfahren ist davon auszugehen, dass das Endprodukt letztlich aufgrund des Einsatzes erheblicher Mengen an Lösungsmitteln eine relativ hohe Restfeuchte aufweist, über die jedoch keine Angaben gemacht werden. Hinzu kommt noch, dass die hergestellten Pellets einen Pankreatin-Gehalt von mehr als 65 bis 85 Gew.-% aufweisen, so dass die Pellets bis zu 15 Gew.-% an Hilfsstoffen und Bindemitteln enthalten können, zumal das Pankreatin von vornherein mit Magnesiumstearat gemischt wird. Das Verfahren ist zudem mehrstufig und von daher aufwendig.

Nach der EP 0 436 110 A1 ist ein Verfahren zur Herstellung von kugelförmigen Pankreatin-Teilchen bekannt, das durch Rotieren einer feuchten Pankreatin-masse zusammen mit einem Lösungsmittel um eine erste Achse und gleichzeitige Verkleinerung der Größe der Masse mit Hilfe von Messern, die um eine zweite Achse rotieren, gekennzeichnet ist, wobei ein Teil des Lösungsmittels entfernt wird, die erste und die zweite Achse stehen dabei in einem Winkel zueinander, wobei die beiden Achsen senkrecht derart zueinander angeordnet sind, dass die Masse einem Winkeleffekt unterworfen wird. Insgesamt wird die Überführung einer bereits entwickelten und verbesserten, jedoch noch lösungsmittelfeuchten Pankreatin-Masse in eine galenische Form offenbart. Als Lösungsmittel dient Aceton. Das offenbarte Verfahren zur Herstellung von Pankreatin-Teilchen sieht die Zerkleinerung der Pankreatin-Masse mit Hilfe von Messern vor, wobei nur ein Teil des Lösungsmittels entfernt wird.

Mit einem magensaftresistenten Film überziehbare Pankreatin-Mikropelletkerne mit einem Pankreatin-Gehalt von 60 bis 85 Gew.-% sind Gegenstand der US 5,378,462. Die Pelletkerne weisen eine kugelige bis ellipsoide Gestalt auf, wobei der Kugeldurchmesser bzw. die kurze Achse im Bereich von 0,7 bis 1,4 mm liegt. Die Pelletkerne sollen eine Partikelgrößenverteilung haben, bei der mindestens 80 % der Pelletkerne ein Verhältnis von kurzer Achse zu langer Achse im Bereich von 1:1 bis 1:2 aufweisen. Damit soll ein verbessertes Verfahren angeboten werden, mit dem neue magensaftresistente filmüberzogene Pankreatin-Mikropellets mit einer hohen Schüttdichte und einer eine gute Passage durch den Pylorus gewährleistenden kleinen Partikelgröße hergestellt werden können.

DE 20 2010 004 926 offenbart ein Pankreatin-Pellet, das ausschliesslich aus Pankreatin gebildet ist und keine Hilfsstoffe oder Bindemittel erhält. Das Pellet hat eine kugelige, ellipsoide oder tropfenförmige Gestalt. Die Pellets werden durch a) Zerkleinerung von vom Schwein oder Rind stammenden Pankreasdrüsen und Unterziehung einer Autolyse b) Gewinnung eines Siebfiltrats c) Ausfällung der Enzyme aus dem Siebfiltrat d) Filtrierung des Gemisches zur Gewinnung des Filterkuchens e) Wärmebehandlung des Filterkuchens bei 80°C oder weniger hergestellt. Der Filterkuchen ist unmittelbar ohne weitere Zusatzstoffe und/oder Bindemittel extrudierbar und sphäronisierbar. Danach werden die erhaltenen Pellets getrocknet. Der wärmebehandelte Filterkuchen weist eine hinreichende Plastizität auf, um eine Extrusion zur Bildung von Strängen aus dem Filterkuchen erreichen zu können. Das Pankreatin-Pellet kann einen Kern bestehend 100% aus Pankreatin und einen Überzug aufweisen. Der Überzug kann eine innere Schicht aus zumindest einem Hilfsstoff und/oder zumindest einem Bindemittel und eine äußere Schicht aus einem magensaftresistenten Material aufweisen.

Aufgabe der vorliegenden Erfindung ist es, Pankreatin-Pellets mit verbesserten Eigenschaften und ein verbessertes und wirtschaftliches Verfahren zu deren Herstellung bereitzustellen, wobei die Pellets zur Vermeidung einer Beeinträchtigung der pharmakologischen Wirkung des Pankreatins ohne zugesetzte Hilfsstoffe, Bindemittel, Zusatzstoffen oder anderweitigen Hilfsstoffen hergestellt werden sollen, um das Pankreatin in möglichst aktiver Form zu erhalten und auch die orale Verabreichung der Pellets erleichtert werden soll. Des Weiteren sollen Pellets mit einem Pankreatin-Gehalt von 100%, mit höherer Pankreatin-Qualität hinsichtlich der Enzymdichte und mit geringster Restfeuchte nach einem Verfahren herstellbar sein, bei dem nur ein einziger Trocknungsvorgang vorgesehen ist, der am Ende des Verfahren vorgenommen wird, um während des Herstellungsvorgangs einwirkende Umgebungsfeuchtigkeit mit erfassen zu können.

Ferner sollen eine pharmazeutische Zusammensetzung, die die erfindungsgemäßen Pankreatin-Pellets enthält, sowie eine Verwendung der Pankreatin-Pellets angegeben werden.

Diese Aufgabe wird durch die Merkmale der Ansprüche 1 und 8 gelöst. Zweckmäßige Ausgestaltungen der Erfindung ergeben sich aus den Merkmalen der Unteransprüche.

Nach Maßgabe der Erfindung sind Pankreatin-Pellets vorgesehen, die ausschließlich aus Pankreatin gebildet sind. Die erfindungsgemäßen Pankreatin-Pellets bestehen somit zu 100 % aus Pankreatin und enthalten keine Hilfsstoffe oder Bindemittel. Die Arzneistoffbeladung betrifft 100%. Die Erfindung umfasst auch Pelletkerne mit einem Pankreatin-Gehalt von 100% mit ausschließlicher Lackierung, wobei auch eine Lackierung möglich ist.

Die erfindungsgemäßen Pankreatin-Pellets, insbesondere Mikropellets, mit einem bevorzugterweise aus dem Pankreas eines Säugetieres gewonnenen Pankreatin und mit einer kugeligen, ellipsoiden oder tropfenförmigen Gestalt sowie einer im Bereich von 0,5 mm bis 2,5 mm liegenden Achse, und mit einem Pankreatin-Gehalt von 100 % sind hergestellt nach folgendem Verfahrensablauf:
a) Zerkleinerung von vom Schwein oder Rind stammenden Pankreasdrüsen und Unterziehung einer Autolyse;
b) Gewinnung eines Enzyme enthaltenden Siebfiltrats durch Filtration des nach Schritt a) erhaltenen Zwischenprodukts;
c) Ausfällung der Enzyme aus dem Siebfiltrat;
d) Filtrierung des nach Schritt c) erhaltenen Gemisches zur Gewinnung des Filterkuchens;
e) Kühlung des nach Schritt d) erhaltenenen Filterkuchens auf -10°C bis -40°C, vorzugsweise auf -30°C zur Erlangung einer hinreichenden Plastizität;
f) Extrudierung des nach Schritt e) gekühlten Filterkuchens unter Ausschluss von Zusatzstoffen und/oder Bindemitteln zur Bildung von Strängen, wobei die extrudierbare Filterkuchenmasse Restfeuchte bzw. Organlösemittelrückstände enthält, die in der Größenordnung von 30 % bis 50 %, insbesondere 40 % liegen;
g) Spheronisation unter Ausschluss von Zusatzstoffen und/oder Bindemitteln oder anderweiten Hilfsstoffen zur Gewinnung von kugeligen, elliptischen oder tropfenförmigen Pellets;
h) Vakuumtrocknung der erhaltenen Pellets bis zum Erhalt einer Restfeuchte von weniger als 3 Gew.-%, vorzugsweise weniger als 1 Gew.-% oder weniger als 0,5 Gew.-%, so dass ein getrocknetes Pankreatinendprodukt mit einer Restfeuchte von weniger als 3 Gew.-% vorzugsweise weniger als 1 Gew.-% oder weniger als 0,5 Gew.-%, erhalten wird.

Die erfindungsgemäßen Pankreatin-Pellets bieten den Vorteil, dass Beeinträchtigungen der pharmakologischen Wirkung des Pankreatins aufgrund des Zusatzes von Hilfsstoffen bzw. Bindemitteln oder beiden vermieden werden.

Unter einem Pellet wird in der vorliegenden Erfindung ein Körper mit einer kugeligen, ellipsoiden oder tropfenförmigen Gestalt verstanden, wobei der Kugeldurchmesser bzw. die kurze Achse im Bereich von 0,5 mm bis 2,5 mm liegt.

Das nach dem erfindungsgemäßen Verfahren hergestellte Pellet, insbesondere Mikropellet weist einen 100 %igen Pankreatingehalt und als besondere Eigenschaft eine Restfeuchte von weniger als 3 Gew.-%, vorzugsweise weniger als 1 Gew.-% oder weniger als 0,5 Gew.-% auf. Die Extrudierung erfolgt ohne Zusatz eines Pelletierhilfsstoffes.

Besonders vorteilhaft ist der Verfahrensschritt e), nach dem eine Kühlung des nach Schritt d) erhaltenen Filterkuchens vorgenommen wird. Die Kühlung ist ein wichtiger Verfahrensschritt, denn sie führt überraschenderweise zur Verbesserung der Strukturviskosität und zur Aufrechterhaltung der plastischen Verformbarkeit des Filterkuchens. Die weitere Verarbeitbarkeit des Filterkuchens im Extruder nach Schritt f) des Verfahrens ist mit Schwierigkeiten verbunden, denn ein nicht gekühlter Filterkuchen weist ein strukturviskoses Verhalten auf, und zwar dahingehend, dass der Filterkuchen unter Scherbeanspruchung eine hohe Fließfähigkeit zeigt und nicht mehr formbar ist ― rheologisch ein plastisches Fließen mit Casson-Charakteristik und teilweiser Rheodestruktion. Diese Strukturviskosität wird überraschender Weise durch Kühlung auf -10°C bis -40°C, vorzugsweise auf -30°C nachhaltig verbessert, so dass auch unter Scherbeanspruchung die gewünschte und erforderliche plastische Verformbarkeit erhalten bleibt. Mit einem Radialextruder mit einer Strangschneidevorrichtung können zylindrische Stränge erzeugt werden, die durch Runden mittels rotierender Scheiben (Spheronizer) zu Pellets formbar sind. Bekannt ist, dass die Pelletherstellung mittels Extrudieren/Spheronisieren zu den (semi-)kontinuierlichen Herstellungsverfahren gehört. Während der Extrusionsschritt kontinuierlich ablaufen kann, erfolgt die Rundung der Extrudate chargenweise in Rundungsmaschinen, wobei Arzneistoffbeladungen bis zu 95 % möglich sind. Hinzu kommt noch, dass der Zusatz eines Pelletierhilfsstoffes beim Extrudieren unverzichtbar sein soll.

Demgegenüber werden nach dem erfindungsgemäßen Verfahren beim Extrudieren überraschenderweise Arzneistoffbeladungen von 100 % erreicht. Pelletierhilfsstoffe werden nicht eingesetzt.

Weitere vorteilhafte Ausgestaltungen der Erfindung sind Gegenstand der Unteransprüche.

Die Erfindung schließt mit ein, dass das Pankreatin-Pellet ein Mikropellet ist.

Das Pankreatin wurde vorzugsweise aus dem Pankreas eines Säugetieres, vorzugsweise aus dem Pankreas eines Schweins oder eines Rindes gewonnen. Die Herstellung der Pellets ist in das Verfahren zur Gewinnung des Pankreatin integriert. Der Zusatz eines gesonderten Hilfsstoffes zur Herstellung einer plastischen Masse, wie dies nach dem Stand der Technik für eine Pelletierung unter Verwendung eines Extruders erforderlich, ist daher nicht notwendig.

Das erfindungsgemäße Verfahren zur Herstellung der Pellets, insbesondere Mikropellets, aus Pankreatin besteht aus folgenden Verfahrensschritten:
a) Zerkleinerung von vom Schwein oder Rind stammenden Pankreasdrüsen und Unterziehung einer Autolyse;
b) Gewinnung eines Enzyme enthaltenden Siebfiltrats durch Filtration des nach Schritt a) erhaltenen Zwischenprodukts;
c) Ausfällung der Enzyme aus dem Siebfiltrat;
d) Filtrierung des nach Schritt c) erhaltenen Gemisches zur Gewinnung des Filterkuchens;
e) Kühlung des nach Schritt d) erhaltenenen Filterkuchens auf -10°C bis -40°C, vorzugsweise auf -30°C zur Erlangung einer hinreichenden Plastizität;
f) Extrudierung des nach Schritt e) gekühlten Filterkuchens unter Ausschluss von Zusatzstoffen und/oder Bindemitteln zur Bildung von Strängen, wobei die extrudierbare Filterkuchenmasse Restfeuchte bzw. Organlösemittelrückstände enthält, die in der Größenordnung von 30 bis 50 %, insbesondere 40 % liegen;
g) Spheronisation unter Ausschluss von Zusatzstoffen und/oder Bindemitteln oder anderweiten Hilfsstoffen zur Gewinnung von kugeligen, elliptischen oder tropfenförmigen Pellets;
h) Vakuumtrocknung der erhaltenen Pellets bis zum Erhalt einer Restfeuchte von weniger als 3 Gew.-%, vorzugsweise weniger als 1 Gew.-%. oder weniger als 0,5 Gew.-%, so dass ein getrocknetes Pankreatinendprodukt mit einer Restfeuchte von weniger als 3 Gew.-% vorzugsweise weniger als 1 Gew.-% oder weniger als 0,5 Gew.-%, erhalten wird.

Der nach dem Verfahrensablauf erhaltene Filterkuchen ist überraschenderweise unmittelbar ohne weitere Zusatzstoffe und/oder Bindemittel extrudierbar und spheronisierbar. Danach werden die erhaltenen Pellets getrocknet. Der Filterkuchen weist eine hinreichende Plastizität auf, um eine Extrusion zur Bildung von Strängen aus dem Filterkuchen erreichen zu können. Nach der Extrusion wird eine Spheronisation vorgenommen, und zwar ebenfalls ohne Zusatz von Zusatzstoffen und/oder Bindemitteln bzw. anderweitigen Hilfsstoffen. Dabei werden kugelige, elliptische oder tropfenförmige Pellets erhalten, die wie sie sind oder als Kerne, die mit einem Überzug versehen werden sollen, verwendet werden können. Danach schließt sich eine Vakuumtrocknung der erhaltenen Pellets bis zum Erhalt einer Restfeuchte von weniger als 3 Gew.-%, vorzugsweise weniger als 1 Gew.-% oder weniger als 0,5 Gew.-%, an. Das so erhaltene Pankreatin-Endprodukt weist somit eine Restfeuchte von weniger als 3 Gew.-%, vorzugsweise weniger als 1 Gew.-% oder weniger als 0,5 Gew.-% auf. Diese sehr geringe Restfeuchte kennzeichnet die Eigenschaft dieser Pellets bzw. Mikropellets und unterscheidet sich somit von den bekannten Pankreatin-Pellets.

Nach einer weiteren erfindungsgemäßen Ausgestaltung besteht das Pellet aus einem Kern mit einem Pankreatin-Gehalt von 100%. Dieser Kern weist keine magensaftresistente Lackierung als Überzug auf; er kann jedoch auch mit einer magensaftresistenten Lackierung versehen sein.

Nach Maßgabe der Erfindung ist somit ein Pankreatin-Pellet vorgesehen, das einen Kern mit oder ohne einen Überzug aufweist, wobei der Kern ausschließlich aus Pankreatin gebildet ist. Der Kern besteht zu 100 % aus Pankreatin und enthält keine Hilfsstoffe bzw. Bindemittel. Hingegen kann der Überzug aus zumindest einem Hilfsstoff und/oder zumindest einem Bindemittel gebildet sein.

Nach einer weiteren Ausführungsform der Erfindung besteht der den Pankreatin-Kern umgebende Überzeug aus einem magensaftresistenten Material. Die hergestellten Pellets werden lackiert, um gegenüber dem Magensaft resistent zu sein und um eine Auflösung erst im Darm zu gewährleisten. Die magensaftresistente Lackierung erfolgt lösungsmittelfrei mit einem Dispersionsfilmbildner auf Polymethacrylsäure-ester-Basis. Die lackierten Pellets aus Siebfraktionen zwischen 0,5 bis 2,5 mm Maschenweite werden in Kapseln konfektioniert.

Die schonende Verarbeitung nach dem erfindungsgemäßen Verfahren zur Herstellung von Pankreatin-Pellets und der Einsatz des reinen, unverdünnten Wirkstoffes führt zu einem Pankreatin-Produkt mit hoher spezifischer Aktivität, das die Herstellung von hochdosierten Pankreatin-Arzneimitteln in relativ kleiner patientenfreundlicher Kapselgröße ermöglicht. Die maximale Kapselfüllmenge für die Masse Pankreatin-Pellets beträgt je nach Kapselgröße 265 mg, 475 mg, 570 mg oder 680 mg.

Nach einer anderen Ausführungsform besteht der Überzug aus einer ersten, inneren Schicht, die den Kern aus Pankreatin umhüllt, und einer zweiten, äußeren Schicht. Vorzugsweise ist die erste Schicht aus zumindest einem Hilfsstoff und/oder zumindest einem Bindemittel gebildet. Die zweite Schicht ist vorzugsweise aus einem magensaftresistenten Material gebildet.

Der Gewichtsanteil von Hilfsstoffen und Bindemitteln an dem Pankreatin-Pellet sollte zwischen 5 und 30 Gew.-% liegen. Der Gewichtsanteil des magensaftresistenten Materials an dem Pankreatin-Pellet sollte zwischen 10 und 30 Gew.-% liegen.

Die Hilfsstoffe bzw. Bindemittel, die den Kern aus Pankreatin umschließen, bewirken einen Zusammenhalt des Kernes beispielweise während der Lagerung und des Transports der Pellets und verhindern damit eine mechanische oder chemische Zerstörung des Kerns. Die verwendeten Hilfsstoffe und Bindemittel müssen pharmakologisch verträglich sein.

Geeignete pharmakologisch verträgliche Hilfsstoffe, die den Überzug oder die innere Schicht des Überzuges, gegebenenfalls gemeinsam mit den nachstehend beschriebenen Bindemitteln bilden können, umfassen zum Beispiel Füllstoffe, Trockenhaltemittel, Gleitmittel, Desintegrationsmittel und Färbemittel. Diese Aufzählung ist nicht abschließend, vielmehr können andere, dem Fachmann bekannte Hilfsstoffe eingesetzt werden.

Beispiele geeigneter Füllstoffe sind aus der Gruppe ausgewählt, die Calciumphosphat, mikrokristalline Cellulose, Dextrane, Dextrin, gefälltes Calciumcarbonat, hydratisiertes Siliciumdioxid, Kaolin, Lactose, Mannitol, Maisstärke, Polyvinylpyrrolidon, Sorbitol, Talkum und Gemische davon umfasst. Beispiele geeigneter Trockenhaltemittel sind aus der Gruppe ausgewählt, die kollodiale Kieselsäure, Talkum und Gemische davon, umfasst. Beispiele geeigneter Desintegrationsmittel sind aus der Gruppe ausgewählt, die, Alginsäure, Amylose, Calciumalginat, Calciumcarbonat, Natriumhydrogencarbonat, quervernetztes Polyvinylpyrrolidon, Kieselsäure, Sagostärke, Stärke und Gemische davon umfasst. Geeignete Gleitmittel, sind aus der Gruppe ausgewählt, die beispielsweise Calcium- oder Magnesiumstearat, Stärke, Stearinsäure, Talkum und Gemische davon umfasst.

Geeignete pharmakologisch verträgliche Bindemittel, die den Überzug oder die innere Schicht des Überzuges, gegebenenfalls gemeinsam mit den vorstehend beschriebene Hilfsstoffen bilden können, sind beispielsweise Verbindungen, die aus der Gruppe ausgewählt sind, die Hydroxypropylmethylcellulose, Polyethylenglycole wie Polyethylenglycol 1500, Polyethylenglycol 2000, Polyethylenglycol 3000, Polyethylenglycol 4000, Polyethylenglycol 6000, Polyethylenglycol 8000, Polyethylenglycol 10000, Polyoxyethylen, Polyoxyethylen-Polyoxypropylen-Copolymere und Gemische davon umfasst. Diese Aufzählung ist nicht abschließend, vielmehr können andere, dem Fachmann bekannte Bindemittel eingesetzt werden. Geeignete Farbstoffe sind beispielweise Lebensmittelfarbstoffe, insbesondere Lebensmittelfarbstoffe, die in der deutschen Arzneimittelfarbstoffverordnung beschrieben sind. Diese Aufzählungen sind nicht abschließend, vielmehr können andere, dem Fachmann bekannte Hilfsstoffe eingesetzt werden.

Die Bildung des Überzuges aus Hilfsstoffen und/oder Bindemitteln bzw. der inneren Schicht des Überzuges kann mittels bekannter Techniken, beispielsweise in einer Wirbelschichtanlage oder einem Kugelcoater, durchgeführt werden. Für eine Beschichtung im Kugelcoater werden die Pellets, die ausschließlich aus Pankreatin bestehen, in den Kugelcoater eingebracht und ein zuvor hergestelltes homogenes Gemisch aus dem Bindemittel und den Hilfsstoffen eingesprüht.

Der magensaftresistente Überzug verhindert eine Zerstörung des Pankreatins im Magen aufgrund der Einwirkung der Magensäure auf die säurelabilen Bestandteile des Pankreatins, insbesondere der Lipasen. Nach der Magenpassage und der Änderung des pH-Wertes beim Eintritt in den Dünndarm löst sich der Schutzfilm, den der magensaftresistente Überzug um den Kern aus Pankreatin gebildet hat, auf, so dass das Pankreatin freigesetzt wird. Das magensaftresistente Material muss bei einem pH-Wert von bis zu 5,5 stabil sein und erst bei einem pH von 5,5 und höher, vorzugsweise 6 und höher die Freisetzung des Pankreatins zulassen.

Geeignete magensaftresistente Materialien, die zur Bildung von Überzügen von Pellets verwendet werden, sind aus dem Stand der Technik bekannt. Üblicherweise enthalten solche Überzüge einen Filmbildner, zumeist einen Weichmacher und in einigen Fällen ein Trennmittel. Geeignete Filmbildner sind beispielsweise aus der Gruppe ausgewählt, die Hydroxypropylmethylcelluloseacetatsuccinat (HPMCAS), Hydroxypropylmethylcellulosephthalat (HPMCP), Celluloseacetatphthalat (CAP), Polyvinylacetatphthalat (PVAP), Methacrylsäure-Methylmethacrylat-Copolymerisate und Methacrylsäure-Ethylacrylat-Copolymerisate sowie Gemische davon umfasst. Diese Aufzählung ist nicht abschließend, vielmehr können andere, dem Fachmann bekannte Filmbildner eingesetzt werden.

Enthält das magensaftresistente Material neben dem Filmbildner einen Weichmacher, so sollte dessen Anteil in Bezug auf den Filmbildner zwischen 1 und 20 Gew.-% liegen. Bevorzugte Weichmacher sind einwertige Alkohole mit 12 bis 30 Kohlenstoffatomen, wie beispielsweise Cetylalkohol (1-Hexadecanol) oder Stearylalkohol (1-Octadecanol) sowie Polyethylenglykole und Triethylcitrat und Gemische davon. Diese Aufzählung ist nicht abschließend, vielmehr können andere, dem Fachmann bekannte Weichmacher eingesetzt werden.

Enthält das magensaftresistente Material neben dem Filmbildner und gegebenenfalls dem Weichmacher auch ein Trennmittel, so sollte dessen Anteil zwischen 0,5 und 5 Gew.-%, bezogen auf den Filmbildner, liegen. Beispielhafte Trennmittel sind Talkum und Dimethicon. Diese Aufzählung ist nicht abschließend, vielmehr können andere, dem Fachmann bekannte Trennmittel eingesetzt werden.

Zur Erzeugung des Überzuges aus dem magensaftresistenten Material können der Filmbilder, gegebenenfalls der Weichmacher und/oder das Trennmittel, in bekannter Art und Weise in einem Lösungsmittel gelöst oder dispergiert werden. Das Lösungsmittel wird nach der Bildung des Überzuges, beispielsweise durch Trocknen, entfernt. Geeignete Lösungsmittel sind aus der Gruppe ausgewählt, die Wasser, Aceton, Alkohole mit 1 bis 5 Kohlenstoffatomen wie Methanol, Ethanol, n- und iso-Propanol, n- und tert-Butanol oder Gemische davon umfasst.

Die Bildung des magensaftresistenten Überzuges kann mittels bekannter Techniken, beispielsweise in einer Wirbelschichtanlage oder einem Kugelcoater, durchgeführt werden. Für eine Beschichtung im Kugelcoater werden die Pellets, entweder unmittelbar die aus 100 % Wirkstoff bestehenden oder die bereits mit dem Überzeug aus den Hilfsstoffen beschichteten Pellets, in den Kugelcoater eingebracht und das magensaftresistente Material aufgesprüht.

Die erfindungsgemäßen Pankreatin-Pellets können eine tropfen- oder kugelförmige Gestalt aufweisen. Vorzugsweise ist dabei der Kern von kugeliger Gestalt, während der Überzug dem Pankretin-Pellet die tropfenförmige Gestalt verleihen kann, was die Dosierung der Pellets, beispielweise aus Tropfenflaschen, erleichtert.

Eine tropfenförmige Gestaltung bietet den Vorteil, dass sich die Pellets beim Fall in einer Richtung orientieren. Dies erlaubt ein einwandfreies Mitzählen der aus einem Vorratsbehälter, wie einer Tropfenflasche, ausgegebenen tropfenförmigen Pankreatin-Pellets.

Die erfindungsgemäßen Pankreatin-Pellets, insbesondere die Mikropellets, sind zur Herstellung von Arzneimitteln geeignet.

Die erfindungsgemäßen Pankreatin-Pellets können zur Herstellung einer pharmazeutischen Zusammensetzung verwendet werden. Vorzugsweise enthält die pharmazeutische Zusammensetzung die erfindungsgemäßen Pankreatin-Pellets in einer pharmakologisch wirksamen Dosis, die für eine orale Verabreichung geeignet ist und für die Behandlung und/oder Prophylaxe von Verdauungsstörungen, akuter Pankreatitis, chronischer Pankreatitis exokriner Pankreasinsuffizienz, Diabetes mellitus insbesondere Typ I und Typ II und zystischer Fibrose vorgesehen ist.

Die erfindungsgemäßen Pankreatin-Pellets sind ebenso zur Herstellung von Nahrungs- oder Lebensmitteln oder als Nahrungsergänzungsmittel geeignet.

Die Erfindung wird nachfolgend anhand von Beispielen, die die Erfindung in keiner Weise beschränken, unter Bezugnahme auf die Zeichnungen näher erläutert. Dabei zeigen:
- Fig. 1: eine Schnittdarstellung einer ersten Ausführungsform eines erfindungsgemäßen Pankreatin-Pellets, das ausschließlich aus Pankreatin gebildet ist;
- Fig. 2: eine Schnittdarstellung einer zweiten Ausführungsform eines erfindungsgemäßen Pankreatin-Pellets mit einem Pankreatin-Kern und einem einlagigen Überzug;
- Fig. 3: eine Schnittdarstellung einer dritten Ausführungsform eines erfindungsgemäßen Pankreatin-Pellets mit einem Pankreatin-Kern und einem zweilagigen Überzug;
- Fig. 4: eine Schnittdarstellung einer vierten Ausführungsform eines erfindungsgemäßen Pankreatin-Pellets mit einem Pankreatin-Kern und einem einlagigen Überzug in Tropfenform; und
- Fig. 5: eine Schnittdarstellung einer fünften Ausführungsform eines erfindungsgemäßen Pankreatin-Pellets mit einem Pankreatin-Kern und einem zweilagigen Überzug in Tropfenform.

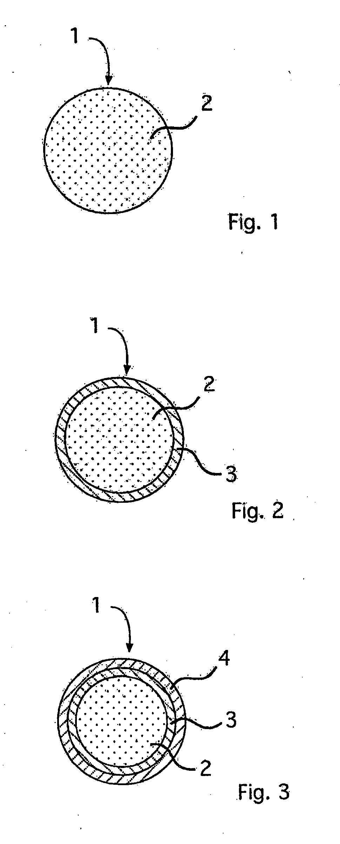

Die in Fig. 1 gezeigte erste Ausführungsform eines erfindungsgemäßen Pankreatin-Pellets 1 lässt erkennen, dass dieses Pellet 1 ausschließlich aus Pankreatin 2 gebildet ist. Es weist keinen Überzug auf.

Im Gegensatz dazu besitzt die in Fig. 2 gezeigte zweite Ausführungsform eines erfindungsgemäßen Pankreatin-Pellets 1 einen Kern 2 und einen einlagigen Überzug 3. Der Kern 2 besteht ausschließlich aus Pankreatin. Der Überzug 3 besteht bevorzugterweise aus einem magensaftresistenten Material. Des Weiteren kann der Überzug Hilfsstoffe, z. B. Bindemittel enthalten. Der Überzug 3 bewirkt einen Zusammenhalt des Pankreatin-Kerns 2 und kann beispielsweise eine mechanische oder chemische Zerstörung des Kerns 2 verhindern und schützt speziell vor dem Angriff der Magensäure.

Die unbeschichteten Pelletkerne sind mechanisch stabil und sind direkt lackierbar.

Die in Fig. 3 gezeigte dritte Ausführungsform des erfindungsgemäßen Pankreatin-Pellets weist einen zweilagigen Überzug auf. Der innere Überzug 3 umhüllt den Kern 2 aus Pankreatin. Auf diese Weise wird der Pankreatin-Kern zusammengehalten und gegen mechanische oder chemische Zerstörung geschützt. Die äußere Lage 4 besteht aus einem magensaftresistenten Material.

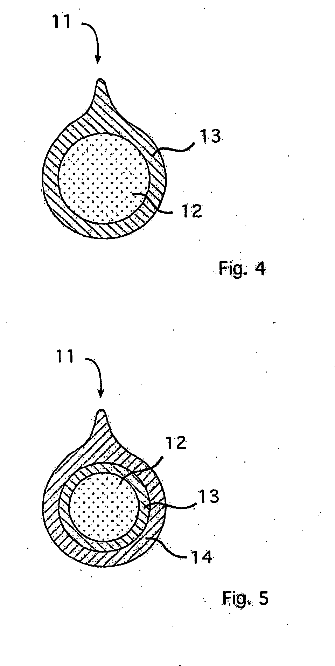

In den Fig. 4 und 5 ist der Überzug tropfenförmig ausgebildet. Bei der in Fig. 4 gezeigten Ausführungsform weist das Pankreatin-Pellet 11 einen einlagigen Überzug 13 in Tropfenform auf, der den kugelförmigen Kern 12, der ausschließlich aus Pankreatin besteht, umhüllt. Der Überzug 13 besteht dabei aus Hilfsstoffen, z. B. Bindemitteln. In Fig. 5 ist der Überzug zweilagig und weist eine innere Schicht 13 und eine äußere Schicht 14 auf. Dabei weist nur die Schicht 14 eine tropfenartige Form auf, während die innere Schicht 13 kugelförmig ist. Die innere Schicht 13 besteht aus Hilfsstoffen, z. B. Bindemitteln, die den Kern 13 zusammenhalten. Die äußere Schicht 14 besteht aus einem magensaftresistenten Material. Die tropfenförmige Ausgestaltung, insbesondere des Pankreatin-Mikropellets ermöglicht u. a. eine individuelle Einzeldosierung von Pellets aus z. B. einer Tropfenflasche.

Das nachstehende Beispiel zeigt eine Möglichkeit zur Herstellung von Pellets aus Pankreatin, bei der zur Extrusion der Filterkuchen einem Kühlvorgang unterworfen wird.

Es wird ausgegangen von einem Lösungsmittel enthaltenden Pankreatin-Filterkuchen, wobei das im Filterkuchen enthaltene Lösungsmittel zu etwa 70% bis 85%, vorzugsweise 75% bis 80% aus Isopropanol oder Aceton besteht, wobei Isopropanol gegenüber Aceton bevorzugt wird, der Rest ist Wasser.

Der feuchte, 40 bis 70 Gew.-% Pankreatin enthaltende Filterkuchen wird zunächst grob zerkleinert, beispielsweise mit Hilfe eines Cutters oder durch schonende Extrusion ohne hohen Druckaufbau durch große Düsen von ca. 5 bis 8 mm Durchmesser, dann auf -10 °C bis -40°C, vorzugsweise auf -30°C, gekühlt. Durch die Kühlung verändert sich die Konsistenz der Masse so, dass sie leicht extrudierbar wird. Außerdem werden die Enzyme des Pankreatins dadurch stabilisiert. Die eigentliche Extrusion erfolgt dann nach bekannten Methoden auf Maschinen wie Schneckenextruder oder Kollergangpresse durch vorzugsweise runde Düsen, deren Durchmesser im Bereich vom 0,5 mm bis 5 mm, vorzugsweise 1,0 mm bis 2,5 mm liegt. Die Extrusionsstränge können, müssen aber nicht, zerschnitten werden. Sie lassen sich direkt mit einer handelsüblichen Rundungsmaschine (Spheronizer) zu spherischen Pellets mit glatter Oberfläche formen. Durch das Erwärmen der Pellets von außen her wird die Oberfläche weicher und verformbar, so dass eine annähernd kugelige (sphärische bzw. sphäroide) Form mit regelmäßiger glatter Oberfläche (ohne Kerben und Zacken) entsteht. Diese Pellets werden dann getrocknet und enthalten nur reines Pankreatin. Die so hergestellten Pellets lassen sich problemlos zu Fertigarzneimitteln weiterverarbeiten durch magensaftresistente Lackierung und z. B. Abfüllung in Hartgelantine-Steckkapseln.

Das erfindungsgemäße Verfahren arbeitet wirtschaftlich, da nur einmal getrocknet wird, weil der bei der Pankreatin-Herstellung feucht anfallende Filterkuchen direkt eingesetzt wird.

Die hohe Wirkstoffdichte, bedingt durch die Vermeidung wiederholten Trocknens mit jeweiligem Enzymverlust und durch den Verzicht auf Hilfs- und Klebstoffe, ermöglicht die Herstellung vergleichbar kleinerer Arzneiformen, die für die Patienten besser schluckbar sind, bzw. bei gleicher Formgröße die gewünschte Applikation höherer Dosen.

### Bezugszeichenliste

- 1: kugelförmiges Pankreatin-Pellet
- 2: Pankreatin
- 3: Überzug aus Hilfsstoffen bzw. Bindemittel
- 4: Überzug aus einem magensaftresistenten Material
- 11: tropfenförmiges Pankreatin-Pellet
- 12: Pankreatin
- 13: Überzug aus Hilfsstoffen und Bindemittel
- 14: Überzug aus einem magensaftresistenten Material

## Patentansprüche

1. Pankreatin-Pellets (1, 11), insbesondere Mikropellets, mit einem bevorzugterweise aus dem Pankreas eines Säugetieres gewonnenen Pankreatin und mit einer kugeligen, ellipsoiden oder tropfenförmigen Gestalt sowie einer, im Bereich von 0,5 mm bis 2,5 mm liegenden Achse und mit einem Pankreatin-Gehalt von 100 %, hergestellt durch:
a) Zerkleinerung von vom Schwein oder Rind stammenden Pankreasdrüsen und Unterziehung einer Autolyse;
b) Gewinnung eines Enzyme enthaltenden Siebfiltrats durch Filtration des nach Schritt a) erhaltenen Zwischenprodukts;
c) Ausfällung der Enzyme aus dem Siebfiltrat;
d) Filtrierung des nach Schritt c) erhaltenen Gemisches zur Gewinnung des Filterkuchens;
e) Kühlung des nach Schritt d) erhaltenen Filterkuchens auf -10°C bis -40°C, vorzugsweise auf -30°C zur Erlangung einer hinreichenden Plastizität;
f) Extrudierung des nach Schritt e) gekühlten Filterkuchens unter Ausschluss von Zusatzstoffen und/oder Bindemitteln zur Bildung von Strängen, wobei die extrudierbare Filterkuchenmasse Restfeuchte bzw. Organlösemittelrückstände enthält, die in der Gröβenordnung von 30 bis 50 %, insbesondere 40 % liegen;
g) Spheronisation unter Ausschluss von Zusatzstoffen und/oder Bindemitteln oder anderweiten Hilfsstoffen zur Gewinnung von kugeligen, elliptischen oder tropfenförmigen Pellets;
h) Vakuumtrocknung der erhaltenen Pellets bis zum Erhalt einer Restfeuchte von weniger als 3 Gew.-%, vorzugsweise weniger als 1 Gew.-%. oder weniger als 0,5 Gew.-%, so dass ein getrocknetes Pankreatin-Endprodukt mit einer Restfeuchte von weniger als 3 Gew.-%, vorzugsweise weniger als 1 Gew.-% oder weniger als 0,5 Gew.-% erhalten wird.

2. Pankreatin-Pellet nach Anspruch 1, **dadurch gekennzeichnet, dass** mit einem Überzug (3, 4) versehen ist.

3. Pankreatin-Pellet nach Anspruch 2, **dadurch gekennzeichnet, dass** der Überzug (3) aus einem magensaftresistenten Material besteht.

4. Pankreatin-Pellet nach Anspruch 2, **dadurch gekennzeichnet, dass** der Überzug (3, 4) aus Pankreatin besteht, oder aus Hilfsstoffen bzw. Bindemitteln gebildet ist.

5. Pankreatin-Pellet nach Anspruch 2, **dadurch gekennzeichnet, dass** der Überzug eine erste, innere Schicht (3), die den Kern (2) aus Pankreatin umhüllt, und eine zweite, äußere Schicht (4) aufweist.

6. Pankreatin-Pellet nach Anspruch 5, **dadurch gekennzeichnet, dass** die erste Schicht (3) aus Hilfsstoffen bzw. Bindemitteln gebildet ist.

7. Pankreatin-Pellet nach Anspruch 5 oder 6, **dadurch gekennzeichnet, dass** die zweite Schicht (4) aus einem magensaftresistenten Material gebildet ist, wobei das Pellet (1, 11) eine tropfen- oder kugelförmige Gestalt aufweist.

8. Verfahren zur Herstellung von aus einem bevorzugterweise aus dem Pankreas eines Säugetieres gewonnenen Pankreatin bestehenden Pellets (1, 11), insbesondere Mikropellets, mit einer kugeligen, ellipsoiden oder tropfenförmigen Gestalt sowie einer im Bereich von 0,5 mm bis 2,5 mm liegenden Achse und mit einem Pankreatingehalt von 100 %, gemäß Anspruch 1 bis 7, **dadurch gekennzeichnet, dass** das Verfahren folgende Schritte umfasst:
a) Zerkleinerung von vom Schwein oder Rind stammenden Pankreasdrüsen und Unterziehung einer Autolyse;
b) Gewinnung eines Enzyme enthaltenden Siebfiltrats durch Filtration des nach Schritt a) erhaltenen Zwischenprodukts;
c) Ausfällung der Enzyme aus dem Siebfiltrat;
d) Filtrierung des nach Schritt c) erhaltenen Gemisches zur Gewinnung des Filterkuchens;
e) Kühlung des nach Schritt d) erhaltenen Filterkuchens auf -10°C bis -40°C, vorzugsweise auf -30°C zur Erlangung einer hinreichenden Plastizität;
f) Extrudierung des nach Schritt e) gekühlten Filterkuchens unter Ausschluss von Zusatzstoffen und/oder Bindemitteln zur Bildung von Strängen, wobei die extrudierbare Filterkuchenmasse Restfeuchte bzw. Organlösemittelrückstände enthält, die in der Gröβenordnung von 30 bis 50 %, insbesondere 40 % liegen;
g) Spheronisation unter Ausschluss von Zusatzstoffen und/oder Bindemitteln oder anderweiten Hilfsstoffen zur Gewinnung von kugeligen, elliptischen oder tropfenförmigen Pellets;
h) Vakuumtrocknung der erhaltenen Pellets bis zum Erhalt einer Restfeuchte von weniger als 3 Gew.-%, vorzugsweise weniger als 1 Gew.-%. oder weniger als 0,5 Gew.-%, so dass ein getrocknetes Pankreatin-Endprodukt mit einer Restfeuchte von weniger als 3 Gew.-% vorzugsweise weniger als 1 Gew.-% oder weniger als 0,5 Gew.-%, erhalten wird.

9. Pharmazeutische Zusammensetzung, **dadurch gekennzeichnet, dass** sie eine pharmakologisch wirksame Menge an Pankreatin-Pellets, gemäß einem der Ansprüche 1 bis 8 enthält.

10. Pharmazeutische Zusammensetzung gemäß Anspruch 1 bis 9, **dadurch gekennzeichnet, dass** sie in einer Dosierungsform vorliegt, die für deren orale Verabreichung geeignet ist.

11. Verwendung von Pankreatin-Pellets, insbesondere Mikropellets, gemäß einem der Ansprüche 1 bis 8 zur Herstellung von Nahrungs- und Lebensmittels oder als Nahrungsergänzungsmittel.

12. Arzneimittel umfassend Pankreatin-Pellets, insbesondere Mikropellets, gemäß einem der Ansprüche 1 bis 8.

13. Arzneimittel gemäß Anspruch 12 zur oralen Verabreichung für die Behandlung und/oder Prophylaxe von Verdauungsstörungen, akuter Pankreatitis, chronischer Pankreatitis, exokriner Pankreasinsuffizienz, Diabetes mellitus, insbesondere Typ I und Typ II und zystischer Fibrose.

## Claims

1. Pancreatin pellets (1, 11), particularly micropellets, with a pancreatin preferably obtained from the pancreas of a mammal and with a spherical, ellipsoidal or drop-shaped form and also an axis lying in the range of 0.5 mm to 2.5 mm and with a pancreatin content of 100 %, produced by:
a) comminuting the pancreatic glands of pigs or cattle and conducting autolysis;
b) obtaining an enzyme-obtaining screen filtrate by filtration of the by-product obtained from step a);
c) precipitating the enzymes from the screen filtrate;
d) filtering the mixture obtained from step c) to obtain the filter cake;
e) cooling the filter cake obtained from step d) to -10 °C to -40 °C, preferably to -30 °C, to obtain sufficient plasticity;
f) extruding the cooled filter cake from step e) in the absence of additives and/or binding agents to form strands, wherein the extrudable filter cake mass contains residual moisture or else organic solvent residues which are in the order of 30 % to 50 %, particularly 40 %;
g) spheronization in the absence of additives and/or binding agents or any other auxiliary substances to obtain spherical, elliptical or drop-shaped pellets;
h) vacuum-drying of the resulting pellets to obtain a residual moisture content of less than 3 % by weight, preferably less than 1 % by weight or less than 0.5 % by weight, so that a dried pancreatin end product with a residual moisture content of less than 3 % by weight, preferably less than 1 % by weight or less than 0.5 % by weight, is obtained.

2. The pancreatin pellet according to claim 1, **characterized in that** it is provided with a coating (3, 4).

3. The pancreatin pellet according to claim 2, **characterized in that** the coating (3) is made of a material resistant to gastric juices.

4. The pancreatin pellet according to claim 2, **characterized in that** the coating (3, 4) is made of pancreatin or of auxiliary substances or binding agents.

5. The pancreatin pellet according to claim 2, **characterized in that** the coating comprises a first, inner layer (3) which encloses the pancreatin core (2) and a second, outer later (4).

6. The pancreatin pellet according to claim 5, **characterized in that** the first layer (3) is formed from auxiliary substances or binding agents.

7. The pancreatin pellet according to claim 5 or 6, **characterized in that** the second layer (4) is formed from a material resistant to gastric juices, wherein the pellet (1, 11) has a drop-shaped or spherical form.

8. A method for producing a pellet (1, 11), particularly micropellets, from pancreatin preferably obtained from the pancreas of a mammal, which pellet has a spherical, ellipsoidal or drop-shaped form and also an axis in the range 0.5 mm to 2.5 mm and a pancreatin content of 100 %, according to claims 1 to 7, **characterized in that** the method comprises the following steps:
a) comminuting the pancreatic glands of pigs or cattle and conducting autolysis;
b) obtaining an enzyme-containing screen filtrate by filtration of the by-product obtained from step a);
c) precipitating the enzymes from the screen filtrate;
d) filtering the mixture obtained from step c) to obtain a filter cake;
e) cooling the filter cake obtained from step d) to -10 °C to -40 °C., preferably to -30 °C, in order to obtain sufficient plasticity;
f) extruding the cooled filter cake from step e) in the absence of additives and/or binding agents to form strands, wherein the extrudable filter cake mass contains residual moisture or else organic solvent residues which are in the order of 30 % to 50 %, particularly 40 %;
g) spheronizing in the absence of additives and/or binding agents or any other auxiliary substances to obtain spherical, elliptical or drop-shaped pellets;
h) vacuum drying the resulting pellets to obtain a residual moisture content of less than 3 % by weight, preferably less than 1 % by weight or less than 0.5 % by weight, so that a dried pancreatin end product with a residual moisture content of less than 3 % by weight, preferably less than 1 % by weight or less than 0.5 % by weight, is obtained.

9. A pharmaceutical compound, **characterized in that** it contains a pharmacologically effective quantity of pancreatin pellets, according to one of the claims 1 to 8.

10. The pharmaceutical compound according to claims 1 to 9, **characterized in that** it exists in a dosage form suitable for the oral administration thereof.

11. Use of pancreatin pellets, particularly micropellets, according to one of the claims 1 to 8 for the production of foodstuffs or as a food additive.

12. A medicine comprising pancreatin pellets, particularly micropellets, according to one of the claims 1 to 8.

13. The medicine according to claim 12 for oral administration for the treatment and/or prevention of digestive disorders, acute pancreatitis, chronic pancreatitis, exocrine pancreatic insufficiency, diabetes mellitus, particularly types I and II, and cystic fibrosis.

## Revendications

1. Boulettes de pancréatine (1, 11) notamment micro-boulettes avec de la pancréatine récupérée de préférence dans le pancréas d'un mammifère et d'une conformation sphérique, ellipsoïdale ou en forme de goutte, ainsi qu'avec un axe de l'ordre de 0,5 à 2,5 mm et avec une teneur en pancréatine de 100 % produite par :
a) Broyage de glandes pancréatiques provenant du porc ou du bovin et soumission de ces dernières à une autolyse ;
b) Extraction d'un produit filtré au tamis par filtrage du produit intermédiaire obtenu après l'étape a),
c) Précipitation des enzymes hors du produit filtré au tamis ;
d) Filtrage du mélange obtenu après l'étape c) pour récolter le gâteau de filtre ;
e) Refroidissement du gâteau de filtre obtenu après l'étape d) à de - 10°C à -40°C, de préférence à -30°C pour obtenir une plasticité suffisante ;
f) Extrusion du gâteau de filtre refroidi obtenu après l'étape e) sous exclusion de matières auxiliaires et/ou d'agents de liaison, pour former des boyaux, la masse de gâteau de filtre extrudable contenant une humidité résiduelle ou des résidus de solvants organiques se situant dans l'ordre de 30 à 50 %, notamment de 40 % ;
g) Sphéronisation sous exclusion de matières auxiliaires et/ou d'agents de liaison ou d'autres adjuvants pour obtenir des boulettes sphériques, elliptiques ou en forme de gouttes ;
h) Séchage sous vide des boulettes obtenues jusqu'à l'obtention d'une humidité résiduelle inférieure à 3 % en poids, de préférence inférieure à 1 % en poids ou inférieure à 0,5 %˙˙en poids, de sorte à obtenir un produit final de la pancréatine avec une humidité résiduelle inférieure à 3 % en poids, de préférence inférieure à 1 % en poids ou inférieure à 0,5 % en poids.

2. Boulette de pancréatine selon la revendication 1, **caractérisée en ce qu'**on munit les boulettes d'un enrobage (3, 4).

3. Boulette de pancréatine selon la revendication 2, **caractérisée en ce que** l'enrobage (3) est constitué d'une matière gastro-résistante.

4. Boulette de pancréatine selon la revendication 2, **caractérisée en ce que** l'enrobage (3, 4) est constitué de pancréatine ou est formé de matières auxiliaires et d'agents de liaison.

5. Boulette de pancréatine selon la revendication 2, **caractérisée en ce que** l'enrobage comporte une première couche (3), interne qui enveloppe le noyau (2) en pancréatine et une deuxième couche (4), externe.

6. Boulette de pancréatine selon la revendication 5, **caractérisée en ce que** la première couche (3) est formée de matières auxiliaires ou d'agents de liaison.

7. Boulette de pancréatine selon 5 ou la revendication 6, **caractérisée en ce que** la deuxième couche (4) est formée d'une matière gastro-résistante, la boulette (1, 11) présentant une conformation en forme de goutte ou sphérique.

8. Procédé de production de boulettes (1, 11) constituées de préférence de pancréatine récupérée dans le pancréas d'un mammifère, d'une conformation sphérique, ellipsoïdale ou en forme de goutte, ainsi qu'avec un axe de l'ordre de 0,5 à 2,5 mm et avec une teneur en pancréatine de 100 % selon les revendications 1 à 7, **caractérisé en ce que** le procédé comporte les étapes suivantes :
a) Broyage de glandes pancréatiques provenant du porc ou du bovin et soumission de ces dernières à une autolyse ;
b) Extraction d'un produit filtré au tamis par filtrage du produit intermédiaire obtenu après l'étape a),
c) Précipitation des enzymes hors du produit filtré au tamis ;
d) Filtrage du mélange obtenu après l'étape c) pour récolter le gâteau de filtre ;
e) Refroidissement du gâteau de filtre obtenu après l'étape d) à de - 10°C à -40°C, de préférence à -30°C pour obtenir une plasticité suffisante ;
f) Extrusion du gâteau de filtre refroidi obtenu après l'étape e) sous exclusion de matières auxiliaires et/ou d'agents de liaison, pour former des boyaux, la masse de gâteau de filtre extrudable contenant une humidité résiduelle ou des résidus de solvants organiques se situant dans l'ordre de 30 à 50 %, notamment de 40 % ;
g) Sphéronisation sous exclusion de matières auxiliaires et/ou d'agents de liaison ou d'autres adjuvants pour obtenir des boulettes sphériques, elliptiques ou en forme de gouttes ;
h) Séchage sous vide des boulettes obtenues jusqu'à l'obtention d'une humidité résiduelle inférieure à 3 % en poids, de préférence inférieure à 1 % en poids ou inférieure à 0,5 %˙˙en poids, de sorte à obtenir un produit final de la pancréatine avec une humidité résiduelle inférieure à 3 % en poids, de préférence inférieure à 1 % en poids ou inférieure à 0,5 % en poids.

9. Composition pharmaceutique, **caractérisée en ce qu'**elle contient une quantité active du point de vue pharmacologique de boulettes de pancréatine selon l'une quelconque des revendications 1 à 8.

10. Composition pharmaceutique selon la revendication 1 à 9, **caractérisée en ce qu'**elle se présente dans un dosage adapté pour une administration orale.

11. Utilisation de boulettes de pancréatine, notamment de micro-boulettes selon l'une quelconque des revendications 1 à 8 pour la production de produits et denrées alimentaires ou en tant que complément alimentaire.

12. Médicament comprenant des boulettes de pancréatine, notamment des micro-boulettes selon l'une quelconque des revendications 1 à 8.

13. Médicament selon la revendication 12 pour l'administration orale, pour le traitement et/ou la prophylaxie de troubles digestifs, de pancréatites aiguës, de pancréatites chroniques, d'insuffisance pancréatique exocrine, du Diabetes mellitus, notamment de type I est de type II et de la fibrose kystique.
